Europäisches Patentamt

European Patent Office

Office européen des brevets

(19)

(11) Publication number: **0 168 171**
**A1**

# (12) EUROPEAN PATENT APPLICATION

(21) Application number: **85304069.9**

(22) Date of filing: **10.06.85**

(51) Int. Cl.⁴: **C 07 C 69/92**
**C 13 D 1/00**

(30) Priority: **11.06.84 US 619085**
**11.06.84 US 619086**
**11.06.84 US 619087**

(43) Date of publication of application:
**15.01.86 Bulletin 86/3**

(84) Designated Contracting States:
**DE FR GB IT**

(71) Applicant: **Velsicol Chemical Corporation**
**341 East Ohio Street**
**Chicago, Illinois 60611(US)**

(72) Inventor: **Luteri, George F.**
**302 S-I-OKA Street**
**Mt. Prospect Illinois 60056(US)**

(72) Inventor: **Stach, Leonard J.**
**372 Southcote Road**
**Riverside Illinois 60546(US)**

(72) Inventor: **Nickell, Louis G.**
**3730 N. Lake Shore Drive**
**Chicago Illinois 60613(US)**

(74) Representative: **Gore, Peter Manson et al,**
**W.P. THOMPSON & CO. Coopers Building Church Street**
**Liverpool L1 3AB(GB)**

(54) 3-Chlorobenzyl-3,6-dichloro-2-methoxybenzoate.

(57) The present invention provides 3-chlorobenzyl-3,6-dichloro-2-methoxybenzoate which may be used as a sugar ripener for sugarcane, citrus and grapes.

EP 0 168 171 A1

-1-

## DESCRIPTION

## 3-CHLOROBENZYL-3,6-DICHLORO-2-METHOXYBENZOATE.

The present invention relates to the new compound, 3-chlorobenzyl-3,6-dichloro-2-methoxybenzoate. It also relates to the use of this new compound to increase the recoverable sugar in sugarcane, citrus and grapes.

In the use of the compound of the present invention to increase the recoverable sugar in sugarcane, sugarcane is normally treated at a late stage of development of the sugarcane wherein most of the sugar formation takes place. Thus, under normal growing conditions and common cultivation practices the active compound of the present invention can be applied to the sugarcane during the period of from about 2 to about 10 weeks before harvesting.

The amount of active compound of the present invention required to effectively increase the recoverable sugar from sugarcane can vary somewhat depending on such factors as the time of application, the weather, crop density, method of application and the like. Generally, an amount of at least 0.56 grams per acre (at least 0.05 pounds per acre) and preferably an amount of from about 0.56 grams per acre to about 56 grams per acre (from about 0.05 pounds per acre to about 5 pounds per acre) can be used. While an amount greater than those mentioned can be used, they will not result in an advantage that would warrant their expense and are, therefore, not practical.

For practical use in treating sugarcane, the active compound of this invention is generally incorporated into compositions or formulations which comprise an inert carrier and an effective amount of the compound. The compositions enable the active compound to be conveniently applied to the sugarcane at the desired rate. The formulations can be liquid formulations such as emulsifiable concentrates or solutions or solid formulations such as dusts, granules or wettable powders.

The preferred compositions are liquid formulations, particularly solutions or emulsifiable concentrates. Emulsifiable concentrates comprise the active compound of the present invention, and as the inert carrier, a solvent and an emulsifier. Such emulsifiable concentrates can be extended with water and/or oil to any desired concentration of active compound for application as sprays to the sugarcane. The emulsifier most commonly used in these concentrates are nonionic or mixtures of nonionic with anionic surface-active agents. With the use of some emulsifier systems an inverted emulsion (water-in-oil) can be prepared.

In addition it has been found that the recoverable sugar content of citrus can be increased by applying to the citrus an effective amount of the compound 3-chlorobenzyl-3,6-dichloro-2-methoxybenzoate.

The term "citrus" includes, but is not limited to, tangerines, oranges, limes, lemons, grapefruit and related plants.

In practising the present invention it is important to realize that the sugar content tends to fluctuate from citrus plant to citrus plant. Therefore, application of the compound will not result the same increase in sugar level in the same amount of time. Generally, the compounds should be applied at from about 4 to about 40 days prior to the intended harvest time. In some plants it may be possible to increase the sugar level by application as little as 4 days prior to harvest, whereas in the majority of cases, application at from about 7 to about 28 days prior to harvest is preferable.

The compounds are applied at a rate of from about 0.7 to about 35 grams per acre (from about 1 to about 50 ounces per acre) about 1.4 to about 3.5 grams per acre (about 2 to about 5 ounces per acre) are optimal. Above 3.5 grams per acre (5 ounces per acre) the increase in effect is nominal, especially in view of the increased cost of the compound applied.

Also it has been found that the recoverable sugar content of grapes can be increased by applying to the grapes or the vines on which they are growing an effective amount of the compound 3-chlorobenzyl-3,6-dichloro-2-methoxybenzoate.

The term "grape" as used herein means the European species Vitis vinifera and selected North American species as well as their hybrids and cultivated varieties and includes, for example, Thompson Seedless, Perlette, Rebeir, Seedless Tokay, Interlocken, Delaware, Tokay, Emperor, Black Corinth, Concord, Himrod, Niagara, Aurore, De Chunac, Chancellor, Delight, Zinfandel, Carignane, Palomino, Petite Sirah, Kyoho, Yaghooti, Chenin Blanc, Cabernet Sauvignon and Sauvignon Blanc.

In practising the present invention it is important to realize that the sugar content tends to fluctuate from season to season and from vine to vine. Therefore, application of the compound will not result the same increase in sugar level in the same amount of time. Generally, the compounds should be applied at from about 4 to about 40 days prior to the intended harvest time. In some vines it may be possible to increase the sugar level by application as little as 4 days prior to harvest, whereas in the majority of cases, application from about 7 to about 28 days prior to harvest is preferable.

The compounds are applied at a rate of from about 0.7 to about 35 grams per acre (from 1 to 50 ounces per acre) and about 1.4 to about 3.5 grams per acre (about 2 to about 5 ounces per acre) are optimal. Above 3.5 grams per acre (5 ounches per acre) the increase in effect is nominal, especially in view of the increased cost of the compound applied.

The compounds are employed in the form of aqueous solutions of dispersions. Generally, where the application device is a spray gun, boom or other device where the solution is expressed through a narrow orifice by pressure, the application rate is 50 to 200 gallons of solution per acre. Where the application is by means of an air sprayer (e.g. a "speed sprayer"), i.e the solution is entrained in a fast moving air stream, more concentrated solutions are employed and about 5 to 50 gallons per acre can be used. Regardless of the amount of solution employed, the pounds of active ingredients per acre should be within the ranges described above.

The compounds are employed in the form of aqueous solutions or dispersions. Generally, where the application device is a spray gun, boom or other device where the solution is expressed through a narrow orifice by pressure, the application rate is 4.47 to 18.71 litres of solution per acre (50 to 200 gallons of solution per acre). Where the application is by means of an air sprayer (e.g. a "speed sprayer"), i.e. the solution is entrained in a fast moving air stream, more concentrated solutions are employed and about 1.87 to 18.71 litres per acre (about 5 to 50 gallons per acre) can be used. Regardless of the amount of solution employed, the amount of active ingredients in grams per acre (ounces or pounds per acre) should be within the ranges described above.

In the aqueous solutions employed for any of sugar cane, citrous and grapes, it is preferred to use a surfactant to prevent the solution from forming globules and "rolling off" upon contact with the leaves of the plant. The surfactant level is generally from 0.1 to 1-1/2% preferred. Suitable surfactants which can be employed include: sorbitan monolaurate; sorbitan monopalmitate; sorbitan monostearate; sorbitan mono-oleate; sorbitan trioleate; polyoxyethylene sorbitan monolaurate; polyoxyethylene sorbitan monopalmitate; polyoxyethylene sorbitan monostearate; polyoxyethylene sorbitan tristearate; polyoxyethylene sorbitan mono-oleate; polyoxyethylene sorbitan trioleate; polyoxyethylene cetyl ether; polyoxyethylene stearyl ether and polyoxyethylene oleyl ether.

The above materials are commonly available under trade names such as "Tween", "Span", "Brij" and "Carbowax". Other surfactants which reduce surface tension can also be employed.

The present invention will now be further described with reference to, but is in no manner limited to, the following Examples, wherein all parts and percentages are by weight unless otherwise specified.

## EXAMPLE 1

### PREPARATION OF 3-CHLOROBENZYL 3,6-DICHLORO-2-METHOXYBENZOATE.

N-chlorobenzyl alcohol (14.2 grams; 0.1 mol); toluene (100 ml); triethylamine (10.1 grams; 0.1 mol) and dicamba acid chloride (24.0 grams; 0.1 mol) were placed in a 250 ml glass, round bottom flask equipped with stirrer, thermometer, and heating mantle and heated to 75°C with stirring for five hours. The mixture was cooled to room temperature overnight and the triethylamine hydrochloride filtered from the solution which was then washed twice with 5% hydrochloric acid and twice with water. After being dried over magnesium sulphate, the toluene was removed and the product recrystallized twice from ethanol. The product had a melting point of 39 - 41°C.

Elemental Analysis:

|  | Theoretical (%) | Found (%) |
|---|---|---|
| C | 52.13 | 52.12 |
| H | 3.21 | 3.18 |
| Cl | 30.78 | 30.67 |

Typical formulations according to the present invention useful for increasing the recoverable sugar in sugarcane and their use are illustrated in the following Examples 2 to 6.

EXAMPLE 2

PREPARATION OF AN EMULSIFIABLE CONCENTRATE

The following ingredients are blended thoroughly until a homogeneous liquid concentrate is obtained. This concentrate is mixed with water to give an aqueous dispersion containing the desired concentration of the active ingredients for use as a spray.

| | |
|---|---|
| 3-Chlorobenzyl-3,6-Dichloro-2-Methoxybenzoate | 25 |
| Sodium lauryl sulfate | 2 |
| Sodium lignin sulfate | 3 |
| Kerosene | 70 |

## EXAMPLE 3

### PREPARATION OF A WETTABLE POWDER.

The following components are mixed intimately in conventional mixing or blending equipment and are then ground to a powder having a particle size of less than about 0.05 mm (about 50 microns). The finished powder is dispersed in water to give the desired concentration of active compound for application to the sugar cane.

| | |
|---|---|
| 3-Chlorobenzyl-3,6-Dichloro-2-Methoxybenzoate | 50 |
| Fuller's earth | 47 |
| Sodium lauryl sulfate | 2.5 |
| Methyl cellulose | 0.5 |

## EXAMPLE 4

### PREPARATION OF A DUST.

The following ingredients are mixed thoroughly and are then ground to an average particle size of less than about 0.05 mm (about 50 microns) to give a dust suitable for application with conventional dusting equipment.

| | |
|---|---|
| 3-Chlorobenzyl-3,6-Dichloro-2-Methoxybenzoate | 10 |
| Powdered talc | 90 |

## EXAMPLE 5

The effectiveness of the compound of the present invention for increasing the recoverable sugar from sugarcane was demonstrated in a field test by applying a solution in acetone diluted for application to the various indicated application rates. The test compound was applied at each rate on the spindle area of each of 20 stalks of sugarcane in a field in Hawaii, using a syringe with a fine needle as the applicator. A set of 10 of these treated stalks from each group was harvested at 4 and 8 weeks after such treatment. In each harvest a set of 10 untreated stalks were also harvested as a control.

The top 14 joints of the treated cane as well as those of the controls were removed, combined and analyzed for juice purity and pol percent cane, following the "press method" developed and described by T. Tanimoto, Hawaiian Planters Record, 57, 133 (1964). Pol percent cane is a polarimetric determination and equals the percentage of sucrose if the latter is the only substance in the solution which will rotate the plane of polarized light. The pol percent cane is a standard method of determining the sucrose content of sugarcane.

The effectiveness of the compound of this invention for increasing the yield of sugar obtained from sugarcane is demonstrated by the data set out in the following Table 1. Each experiment represents a separate test conducted at a different time. The cane was harvested 8 weeks after application of the test compound.

TABLE 1.

| Experiment Number | | | Rate of Application | | Pol % Cane | Juice Purity |
|---|---|---|---|---|---|---|
| | | | grams/ acre | pounds/ acre | | |
| 1 | | Test Compound | 11.21 | (1.0) | 12.68 | 86.04 |
| | | Control | 1 | (0) | 11.48 | 82.21 |
| 2 | | Test Compound | 11.21 | (1.0) | 15.07 | 89.31 |
| | | Control | 0 | (0) | 9.22 | 72.88 |
| 3 | | Test Compound | 1.12 | (0.1) | 10.87 | 79.52 |
| | | Test Compound | 0.56 | (0.05) | 11.87 | 82.87 |
| | | Control | 0 | (0) | 9.42 | 77.58 |
| 4 | | Test Compound | 5.61 | (0.5) | 13.47 | 87.15 |
| | | Test Compound | 1.12 | (0.1) | 12.84 | 85.58 |
| | | Control | 0 | (0) | 9.52 | 77.12 |
| 5 | | Test Compound | 5.61 | (0.5) | 13.97 | 86.52 |
| | | Test Compound | 1.12 | (0.1) | 13.26 | 84.77 |
| | | Control | 0 | (0) | 9.98 | 77.32 |
| 6 | | Test Compound | 5.61 | (0.5) | 13.22 | 83.82 |
| | | Test Compound | 1.12 | (0.1) | 13.40 | 84.44 |
| | | Control | 0 | (0) | 11.61 | 80.66 |
| 7 | | Test Compound | 11.21 | (1.0) | 13.33 | 84.92 |
| | | Test Compound | 5.61 | (0.5) | 14.21 | 84.30 |
| | | Test Compound | 1.12 | (0.1) | 12.00 | 81.53 |
| | | Control | 0 | (0) | 9.53 | 76.14 |
| 8 | | Test Compound | 11.21 | (1.0) | 12.56 | 83.23 |
| | | Test Compound | 5.61 | (0.5) | 14.63 | 88.31 |
| | | Test Compound | 1.12 | (0.1) | 12.96 | 85.97 |
| | | Control | 0 | (0) | 11.20 | 81.15 |

### EXAMPLE 6

In order to demonstrate the unique properties of the present compound, tests were performed on sugarcane plants in accordance with the foregoing procedures using other substituted benzyl esters of dicamba. The results of these tests in comparison to the results obtained using the compound of the present invention are reported in the following Table 2 using the following test scale:

- = Negative effect

0 = No effect

1 = Low level effect

2 = Moderate effect

3 = Commercial level effect

4 = Above commercial level effect

N.T. = No test

### TABLE 2

| Substituent | Position of Substituent | | |
|---|---|---|---|
| | Ortho | Meta | Para |
| Nitro | 1* | - | -* |
| Chloro | 2* | 4 | 0* |
| Bromo | 0 | - | - |
| Fluoro | NT | NT | 1 |
| Methyl | 2 | 0 | - |
| Methoxy | 0 | 2 | - |

The results represent two tests for each compound except for the compounds identified with an * which represents three tests and the claimed compound (meta-chloro) which represents 30 tests.

### EXAMPLE 7

In order to demonstrate the usefulness of 3-chlorobenzyl-3,6-dichloro-2-methoxybenzoate in increasing the recoverable sugar in citrus, tests were

-11-  0168171

performed on Satsuma Mandarin Oranges. In the tests the results are shown by the Brix readings, colouring and acid content. The Brix value is determined by randomly filtering if necessary to remove solids, and "reading" the juice with a hand refractometer to determine the % Brix. The tests were performed on Satsuma Mandarin Oranges. The results of these experiments are as follows:

### TABLE 3

| Concentration (ppm) | Frequency of Spraying | Colouring | Brix | Acids |
|---|---|---|---|---|
| 50 | 1 | 7.3 | 10.0 | 0.71 |
| " | 2 | 7.1 | 10.3 | 0.76 |
| 100 | 1 | 7.0 | 10.1 | 0.73 |
| " | 2 | 8.3 | 10.2 | 0.71 |
| Control | | 6.2 | 9.2 | 0.78 |

### EXAMPLE 8

In order to demonstrate the usefulness of 3-chlorobenzyl-3,6-dichloro-2-methoxybenzoate in increasing the recoverable sugar in grapes, tests were performed on two varieties of grapes: Thompson Seedless and Carignane. In the tests the results are shown by the Brix readings. This value is determined by randomly selecting one or more berries from a bunch of grapes, squeezing out the juice, filtering if necessary to remove solids, and "reading" the juice with a hand refractometer to determine the % Brix. The method is described generally in the publication by the California Department of Food and Agriculture entitled "Fruit and Vegetable Standardization", Title III, Article 25, sec. 1436.5 (Register 75, No.4, 1-25-75). The results of these experiments are as follows:

0168171

## TABLE 4

### THOMPSON SEEDLESS GRAPES.

| Rate of Application | | Brix Value Days After Application | |
|---|---|---|---|
| grams per acre | (ounces per acre) | 8 | 13 |
| 0.35 | 0.5 | 19.65 | 19.06 |
| 0.7 | 1.0 | 19.73 | 19.97 |
| 1.4 | 2.0 | 20.62 | 20.44 |
| 0 | 0 | 18.63 | 18.38 |

## TABLE 5

### CARIGNANE GRAPES.

| Rate of Application | | Brix Value Days After Application | | |
|---|---|---|---|---|
| grams per acre | (ounces per acre) | 15 | 30 | 37 |
| 0.35 | 0.5 | 19.4 | 20.7 | 22.0 |
| 0.7 | 1.0 | 18.55 | 20.4 | 20.2 |
| 1.4 | 2.0 | 18.7 | 19.5 | 20.5 |
| 0 | 0 | 17.3 | 18.7 | 19.2 |

. . . . . . . . . . . . . . . . . . . . . . . . . . . . . . . . . . . . . . . . . . . . . . . . . . .

CLAIMS

1. 3-Chlorobenzyl-3,6-dichloro-2-methoxybenzoate.

2. A method for increasing the recoverable sugar contained in sugarcane characterized in that the sugarcane plant is contacted with an effective amount of 3-chlorobenzyl-3,6-dichloro-2-methoxybenzoate.

3. A method according to claim 2, wherein the sugarcane is contacted with from about 0.56 to about 56 grams per acre (from about 0.05 to about 5 pounds per acre) of 3-chlorobenzoyl-3,6-dichloro-2-methoxybenzoate.

4. A method according to claim 2 or 3 wherein the sugarcane is contacted with 3-chlorobenzyl-3,6-dichloro-2-methoxybenzoate during the period of from about 2 to about 10 weeks before harvest.

5. A method for increasing the recoverable sugar in citrus characterized in that there is applied to a citrus plant an effective amount of 3-chlorobenzyl-3,6-dichloro-2-methoxybenzoate.

6. A method according to claim 5, wherein the citrus is selected from oranges, grapefruit, lemons and tangerines.

7. A method of increasing the recoverable sugar in grapes characterized in that there is applied to the grapes or the vines on which they are growing an effective amount of the compound of claim 1.

8. A method according to any of claims 5 to 7, wherein the amount of 3-chlorobenzyl-3,6-dichloro-2-methoxybenzoate applied to the citrus plant, grapes or the vines on which the grapes are growing is from about 0.7 to about 35 grams per acre (from about 1 to about 50 ounces per acre).

9. A method according to claim 8, wherein the amount of 3-chlorobenzyl-3,6-dichloro-2-methoxybenzoate applied is from about 1.4 to about 3.5 grams per acre (about 2 to about 5 ounces per acre).

10. A method according to any of claims 5 to 9, wherein the 3-chlorobenzyl-3,6-dichloro-2-methoxybenzoate is applied to the citrus plant, grapes or the vines on which the grapes are growing at from about 4 to about 40 days prior to harvest.

11. A method as claimed in claim 10, in which the 3-chlorobenzyl-3,6-dichloro-2-methoxybenzoate is applied at from about 7 to about 28 days prior to harvesting.

12. A composition for increasing the recoverable sugar contained in sugar cane, citrus or grapes, characterized by 3-chlorobenzyl-3,6-dichloro-2-methoxybenzoate and an inert carrier.

13. A composition according to claim 12, wherein the inert carrier is a solvent and an emulsifier so that the composition is an emulsifiable concentrate.

. . . . . . . . . . . . . . . . . . . . . . . . . . . . . . . . . . . . . . . . . . . . . . . . . . . . . .

# European Patent Office

## EUROPEAN SEARCH REPORT

## DOCUMENTS CONSIDERED TO BE RELEVANT

EP 85304069.9

| Category | Citation of document with indication, where appropriate, of relevant passages | Relevant to claim | CLASSIFICATION OF THE APPLICATION (Int Cl 4) |
|---|---|---|---|
| A | DE - B - 1 417 429 (VELSICOL)<br>* Claim 4; column 5, line 20 - column 8 * | 1-13 | C 07 C 69/92<br>C 13 D 1/00 |
| A | DE - A - 2 104 102 (VELSICOL)<br>* Claims; examples * | 1-4,12, 13 | |
| A | GB - A - 1 021 893 (FISONS PEST CONTROL)<br>* Page 2, lines 100-106; examples 1-3; claims * | 1-4,12, 13 | |

|  |
|---|
| TECHNICAL FIELDS SEARCHED (Int Cl 4) |
| C 07 C 69/00 |
| C 07 C 65/00 |
| C 13 D |

The present search report has been drawn up for all claims

| Place of search | Date of completion of the search | Examiner |
|---|---|---|
| VIENNA | 06-09-1985 | HOFBAUER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82